# EUROPEAN PATENT SPECIFICATION

(11) **EP 3 735 267 B1**
(45) Date of publication and mention of the grant of the patent: **14.09.2022**
(21) Application number: 19705386.1
(22) Date of filing: 04.01.2019
(51) Int. Cl.: A61K 39/00, A61K 31/4745, A61K 31/513, A61K 31/519, A61K 31/555, A61P 35/00

(54) **ADMINSITRATION OF MULTIPLE BOLUSES OF [6R] MTHF IN A 5-FLUOROURACIL-BASED CHEMOTHERAPY**
[6R]-MTHF-BOLUS-MEHRFACHVERABREICHUNG IN 5-FLUORURACIL-BASIERTER CHEMOTHERAPIE
ADMINISTRATION DE BOLUS MULTIPLES [6R]-MTHF DANS UNE CHIMIOTHÉRAPIE À BASE DE 5-FLUOROURACILE

(30) Priority: 05.01.2018 WO PCT/EP2018/050274; 03.04.2018 US 201862651910 P; 19.11.2018 US 201862769289 P
(43) Date of publication of application: 11.11.2020
(73) Proprietor: Isofol Medical AB, 413 46 Göteborg (SE)
(72) Inventor: Ganlöv, Karin, 411 25 Göteborg (SE); Östberg, Magnus, 416 53 Göteborg (SE); Lindberg, Per L., 413 01 Göteborg (SE); Sundén, Gunnel E., 41301 Gothenburg (SE); Gustavsson, Bengt, 426 79 Vastra Frolunda (SE)
(74) Representative: AWA Denmark A/S
(86) International application number: PCT/IB2019/000008
(87) International publication number: WO 2019/135157

(56) References cited:
- WO-A2-2005/097086
- WO-A2-2007/064968
- Muhammad Wasif Saif ET AL: "Phase III Multicenter Randomized Clinical Trial to Evaluate the Safety and Efficacy of CoFactor / 5-Fluorouracil/Bevacizumab Versus Leucovorin/ 5-Fluorouracil/Bevacizumab as Initial Treatment for Metastatic Colorectal Carcinoma", Clinical Colorectal Cancer, 1 September 2006 (2006-09-01), pages 229-234, XP055358951, Retrieved from the Internet: URL:http://www.clinical-colorectal-cancer. com/article/S1533-0028(11)70289-4/pdf [retrieved on 2017-03-27]

## Description

The instant application claims priority to PCT/EP2018/050274 filed January 5, 2018, U.S. Provisional Application Serial No. 62/651,910 filed April 3, 2018, and U.S. Provisional Application Serial No. 62/769,289 filed November 19, 201 8.

### FIELD OF THE INVENTION

The present invention relates to the treatment of solid tumors in humans diagnosed with colorectal cancer and metastatic colorectal cancers in a chemotherapeutic regimen comprising the administration of [6R]-5,10-methylene tetrahydrofolate (6R-MTHF), 5-fluorouracil (5-FU), bevacizumab, and oxaliplatin. Any references in the description to methods of treatment refer to the compounds, pharmaceutical compositions and medicaments of the present invention for use in a method for treatment of the human (or animal) body by therapy.

### BACKGROUND OF THE INVENTION

Patients diagnosed with colorectal cancer (CRC) or metastatic colorectal cancers (mCRC) are usually treated surgically and, in most circumstances, with curative intent. Surgery, in fact, remains the primary modality of treatment for malignancies of the lower gastrointestinal tract, and standard resection is the only therapy required for early-stage cancer. As the stage of the tumor advances, in terms of depth of penetration and lymph node involvement, the chance of cure with surgery alone diminishes and the rate of local recurrence increase. In such cases, surgery may either be combined with adjuvant treatment or be performed for palliative control of symptoms only.

5-fluorouracil (5-FU) was first introduced in 1957, and still remains an essential part of the treatment of a wide range of solid tumours such as breast tumours, tumours of head and neck and gastrointestinal tumours. 5-FU in combination with the folate leucovorin (LV)(the calcium salt of 5-formyl-5,6,7,8-tetrahydrofolic acid or calcium folinate) has been the cornerstone in the treatment of colorectal cancer and metastatic colorectal cancers for decades. Standard first-line systemic therapy for metastatic colorectal cancer, in accordance with current European Society for Medical Oncology (ESMO) guidelines, is chemotherapy with 5-FU, LV, oxaliplatin/irinotecan and bevacizumab. Van Custem et al. (2016) ESMO Consensus Guidelunes for the Management of Patients with Metastatic Colorectal Cancer. Annals of Oncology 27: 1386-1422.

The overall response rate of 5-FU alone is quite limited, reaching levels of 10-15 % [Johnston P.G., Kaye S. Capcetabine; a novel agent for the treatment of solid tumours. Anticancer Drugs 2001, 12: 639-646] and modulation strategies to increase the anticancer activity of 5-FU have been developed. One of the most widely used strategies is a coadministration of Leucovorin, the calcium salt of folinic acid. Leucovorin (LV) acts as a stabiliser of the ternary complex, a structure formed by ¹⁾ 5,10-methylene tetrahydrofolate, the active metabolite of LV, ²⁾ FdUMP, the 5-FU active metabolite and ³⁾ Thymidylate synthase. This ternary complex inhibits the enzyme thymidylate synthase, an enzyme necessary for DNA synhesis [Longley D.B. et al. 5-Fluorouracil. Mechanisms of action and clinical strategies, Nat Rev Cancer. 2003 May;3(5):330-8. Review]. By adding LV to 5-FU the overall response rates increased to over 20 % [Longley D.B. et al. 2003 *ibid.*]*.*

5-FU-based regimens are commonly used in the treatment of solid tumors of the gastrointestinal tract. For colorectal tumors, the original response rate for 5-FU given as a monotherapy was only around 10%. By adding Leucovorin (LV) the response rate was improved to 21% [Thirion P, Michiels S, Pignon JP, Buyse M, Braud AC, Carlson RW, O'Connell M, Sargent P, Piedbois P (2004) Modulation of fluorouracil by leucovorin in patients with advanced colorectal cancer: an updated meta-analysis. J Clin Oncol 22(18):3766-3775]. However, LV needs to be converted to the active metabolite [6R]-5,10-methylenetetrahydrofolate (methyleneTHF), which subsequently forms a ternary complex with deoxyuridine monophosphate (dUMP) and the target enzyme thymidylate synthase (TS) in a reaction where dUMP is converted to dTMP [Jarmula A, Cieplak P, Montfort WR (2005) 5,10-Methylene-5,6,7,8-tetrahydrofolate conformational transitions upon binding to thymidylate synthase: molecular mechanics and continuum solvent studies. J Comput Aided Mol Des 19(2):123-136]. This reaction is inhibited when the fluorinated metabolite of 5-FU, FdUMP, binds the complex instead of dUMP [Parker WB, Cheng YC (1990) Metabolism and mechanism of action of 5-fluorouracil. Pharmacol Ther 48(3):381-395]. As such, LV does not have antitumoral effect, but enhances the effect of 5-FU by providing methyleneTHF in abundance, which stabilizes the ternary complex [Porcelli L, Assaraf YG, Azzariti A, Paradiso A, Jansen G, Peters GJ (2011) The impact of folate status on the efficacy of colorectal cancer treatment. Curr Drug Metab 12(10):975-984]. The inhibition impacts cells with a high proliferation rate most, such as tumor epithelial cells. This in turn leads to suppression of DNA synthesis in the cells, which may lead to cell death by apoptosis.

The required metabolic activation of LV into methylene THF is likely to lead to interindividual differences, which may be the reason the response rate for 5-FU given as a monotherapy was only improved to 21%. Adjuvant therapies have been shown to improve treatment outcome in metastatic CRC with prolonged survival [Cunningham D, Atkin W, Lenz HJ, Lynch HT, Minsky B, Nordlinger B, et al. Colorectal cancer. Lancet. 2010 Mar 20;375(9719):1030-47]. Standard first-line adjuvant therapy of CRC includes single and combination chemotherapy with the agent 5-Fluorouracil (5-FU) [Cunningham D (2010)]. Treatment with 5-FU is usually given in combination with high doses of folate (or Leucovorin, LV) which significantly enhances the therapeutic effect of 5-FU in metastatic colorectal carcinoma. In fact, modulation of 5-FU with LV in metastatic disease has shown prolongation of the time-to-progression (TTP) of disease [Petrelli N, Douglass HO Jr, Herrera L, Russell D, Stablein DM, Bruckner HW, et al. The modulation of fluorouracil with leucovorin in metastatic colorectal carcinoma: a prospective randomized phase III trial. Gastrointestinal Tumor Study Group. J Clin Oncol. 1989 Oct;7(10):1419-26].

A reduced folate, fotrexorin calcium (CoFactor^{®}) ((*dl*)-5,10,-methylenepteroyl-monoglutamate calcium salt, or [6R,S]-5,10-methylene-THF Ca salt), also known as racemic methyleneTHF, has been suggested as an alternative to LV based on the assumption that direct administration of the reduced folate methylene THF in place of LV might offer significant advantages with respect to clinical activity. CoFactor^{®} is a 1:1 mixture of the two diastereoisomers [Odin, E., Carlsson, G., Frosing, R., Gustavsson, B., Spears, C.P., Larsson, P.A., 1998. Chemical stability and human plasma pharmacokinetics of reduced folates. Cancer Invest. 16, 447-455]. As the [6R]-isomer is the directly active co-substrate of TS, it was anticipated that the administration of CoFactor^{®}, instead of leucovorin, would be advantageous due to lower inter- and intrapatient variability regarding both clinical safety and efficacy.

Indeed, in a Phase II Trial in previously untreated metastatic colorectal cancer, the overall response rate for CoFactor^{®} was found to be 35% [Saif, M.W, Merritt, J, Robbins J, Stewart J., Schupp, J, 2006. Phase III Multicenter Randomized Clinical Trial to Evaluate the Safety and Efficacy of CoFactor®/5-Fluorouracil/Bevacizumab Versus Leucovorin/5-Fluorouracil/ Bevacizumab as Initial Treatment for Metastatic Colorectal Carcinoma Clinical Colorectal Cancer, Vol. 6, No. 3, 229-234, 2006], and in another phase I/II clinical trial it was demonstrated that CoFactor^{®} combined with 5-FU showed clinical benefit in pancreas cancer, defined as stable disease or tumor response, in 40% of patients [Saif, M.W., Makrilia N., Syrigos K., 2010. CoFactor: Folate Requirement for Optimization of 5-Fluouracil Activity in Anticancer Chemotherapy. Journal of Oncology Vol. 1-5]. However, apart from presenting an unnecessary hepatic detoxification burden, the unnatural (6S)-isomer is a partial competitive inhibitor of the natural [6R]-isomer regarding its effect as co-substrate for TS [Leary, R.P., Gaumont, Y., Kisliuk, R.L., 1974. Effects of the diastereoisomers of methylenetetrahydrofolate on the reaction catalyzed by thymidylate synthetase. Biochem. Biophys. Res. Commun. 56, 484-488]. Furthermore, in a Phase IIb study CoFactor^{®} in colorectal cancer was not demonstrated to be more efficacious than leucovorin as no significant differences between the study arms with regard to either efficacy or safety could be found, and a planned Phase III study colorectal cancer was discontinued before completion [Press release: ADVENTRX Provides Update on Cofactor Program. Nov 2, 2007]. Consequently, there remains a need for an improved folate-enhanced 5-FU treatment protocol.

### SUMMARY OF THE INVENTION

The inventors have discovered methods of treating patients diagnosed with colorectal cancer or metastatic colorectal cancers comprising the administration of [6R]-5,10-methylene tetrahydrofolate (6R-MTHF), 5-fluorouracil (5-FU), bevacizumab, and oxaliplatin. These methods surprisingly reduce disease progression, maintain stable disease and provide improved overall response rates compared to standard treatments that utilize folates such as folinic acid.

In one embodiment, the present invention provides methods for treating a patient diagnosed with colorectal cancer or metastatic colorectal cancer comprising the steps of:
a) administering to said patient a pharmaceutical composition comprising bevacizumab at a dose of 5 mg/kg bevacizumab by intravenous infusion;
b) followed by administering to said patient a pharmaceutical composition comprising oxaliplatin at a dose of 85 mg/m² oxaliplatin by intravenous infusion;
c) followed by administering to said patient a pharmaceutical composition comprising 5-fluorouracil (5-FU) at a dose of 400 mg/m² 5-FU by intravenous bolus;
d) followed by administering to said patient a pharmaceutical composition comprising a pharmaceutically acceptable salt of a folate, 6R-MTHF, at a dose of 60 mg/m² 6R-MTHF by intravenous bolus;
e) followed by administering a pharmaceutical composition comprising 5-fluorouracil (5-FU) at a dose of 2,400 mg/m² 5-FU by intravenous infusion over 46 hours; and
f) administering a pharmaceutical composition comprising a pharmaceutically acceptable salt of 6R-MTHF at a dose of 60 mg/m² 6R-MTHF by intravenous bolus.

The pharmaceutical compositions of 6R-MTHF, 5-FU, bevacizumab, and oxaliplatin are administered intravenously either as an intravenous infusion or as a bolus. As used herein, "intravenous bolus" means a method of intravenous administration wherein a single dose of a pharmaceutical composition is given all at once. In one embodiment, administration by intravenous bolus is completed within 10 minutes. In a preferred embodiment, administration by intravenous bolus is completed within 5 minutes. In a preferred embodiment, administration by intravenous bolus is completed within 3 minutes. In a preferred embodiment, administration by intravenous bolus is completed within 2 minutes. In a preferred embodiment, administration by intravenous bolus of steps (c), (d) and (f) is completed within 10, 5, 3 or 2 minutes.

In one embodiment, administration by intravenous bolus of 6R-MTHF of steps (d) and (f) are at a dose of 30, 60 or 120 mg/m². In a preferred embodiment, the administration by intravenous bolus of 6R-MTHF of steps (d) and (f) is at a dose of 60 mg/m².

In contrast, as used herein, "intravenous infusion" refers to intravenous infusion of a single dose given over at constant concentration over a period of time. For example, administration by intravenous infusion is completed in at least 30 minutes, 60, 90, 120, or 180 minutes. In another example, administration by intravenous infusion of step (e) is conducted over a period 46 hours.

In certain embodiments, the intravenous bolus administration of step (d) follows completion of the intravenous bolus administration of step (c) by up to 30, 60, 90 or 120 minutes. In another embodiment, the intravenous bolus administration of step (f) follows initiation of the intravenous infusion of step (e) by up to 30, 60, 90 or 120 minutes. In yet another embodiment, the intravenous bolus administration of step (f) is completed within 30 or 60 minutes of the initiation of step (d). In another embodiment, the intravenous bolus administration of step (f) follows initiation of step (c) by up to 30, 60, 90, 120 or 180 minutes.

In one embodiment the intravenous bolus administration of step (f) may be initiated before the initiation of the intravenous infusion of step (e). In another embodiment, the intravenous infusion of step (e) is paused during the intravenous bolus administration of step (f).

The method of treating patients diagnosed with colorectal cancer and metastatic colorectal cancers comprising steps (a) to (f) may be repeated about every two weeks. As used herein, "about every two weeks" means between 10 and 18 days. In one embodiment, the methods disclosed herein may be repeated every two weeks.

The pharmaceutical compositions of [6R]-5,10-methylene tetrahydrofolate (6R-MTHF) used in the methods disclosed herein, are provided as pharmaceutically acceptable salt. Examples of pharmaceutically acceptable salts include, but are not limited to, mineral or organic acid salts of basic residues such as amines; alkali or organic salts of acidic residues such as carboxylic acids; and the like. In one embodiment, the pharmaceutically acceptable salt of 6R-MTHF is the sulfate salt. In one embodiment, the pharmaceutically acceptable salt of 6R-MTHF is the hemisulfate salt. In certain embodiments, the pharmaceutically acceptable salts of 6R-MTHF used in the methods disclosed herein may be provided as a lyophilisate. In another embodiment, the lyophilisates used in the methods disclosed herein are prepared from 6R-MTHF hemisulfate salt. In a preferred embodiment, the lyophilisate used in the methods disclosed herein is prepared from a 6R-MTHF hemisulfate salt and further comprises other salts and excipients and in one embodiment trisodium citrate dihydrate.

In certain embodiments, the lyophilisates used in the methods disclosed herein are reconstituted in aqueous media prior to use. In one embodiment, the aqueous media is water or saline. In a preferred embodiment, the aqueous media is water. In other embodiments, the lyophilisate comprises 100 mg of 6-MTHF. In a preferred embodiment, the lyophilisate comprising 100 mg 6R-MTHF is reconstituted by the addition of 10 mL water. In yet another embodiment, the lyophilisate comprising 100 mg of 6R-MTHF is provided in a container. In another embodiment of the methods disclosed herein, the reconstituted lyophilisate forms a solution with a concentration of between 5 and 20 mg/mL 6R-MTHF. In a preferred embodiment, the reconstituted lyophilisate forms a solution with a concentration of between 7 and 12 mg/mL 6R-MTHF. More preferably, the reconstituted lyophilisate forms a solution with a concentration of 10 mg/mL 6R-MTHF. In other embodiments of the methods disclosed herein, the reconstituted lyophilisate is isotonic. In yet another embodiment, the reconstituted lyophilisate has a pH of between 7.0 and 9.0, more preferably between pH 8.0 and 9.0, most preferably between 8.3 and 8.7 and even more preferably pH 8.5.

The 6R-MTHF or pharmaceutically acceptable salts thereof has a diastereomeric purity of at least 98% d.e., preferably 99% d.e., more preferably 99.8% d.e. and most preferably 99.9% d.e.

In one embodiment, the methods of treating colorectal cancer and metastatic colorectal cancer is practiced on a patient diagnosed with colorectal cancer or metastatic colorectal cancer having at least one solid tumor. In another embodiment, the colorectal cancer is colorectal adenocarcinoma and in yet another embodiment the colorectal cancer or colorectal adenocarcinoma is verified by biopsy.

In one embodiment, the methods of treating colorectal cancer and metastatic colorectal cancer reduces the size of one or more solid tumors compared to the one or more solid tumors before treatment or compared to "baseline size", as that term is defined herein. In another embodiment, the methods disclosed herein reduce the size of one or more solid tumors after 2, 4, 8, 16 or 24 weeks of treatment after repeating steps (a) to (f) about every two weeks. In another embodiment, the size of the one or more solid tumors is evaluated by computerized axial tomography (CAT or CT) or magnetic resonance imaging (MRI) at 8, 16, or 24 weeks after repeating steps (a) to (f) about every two weeks.

In yet another embodiment, the solid tumor has a greater reduction in size (relative to untreated tumor or "baseline size", as that term is defined herein) compared to treatment of solid tumors in a patient diagnosed with colorectal cancer or metastatic colorectal cancer treated by administering at least bevacizumab, oxaliplatin, 5-FU and folinic acid (LV), wherein the administering of at least bevacizumab, oxaliplatin, 5-FU and LV comprises the steps of:
a. administering to said patient a pharmaceutical composition comprising bevacizumab at a dose of 5 mg/kg bevacizumab by intravenous infusion;
b. followed by administering to said patient a pharmaceutical composition comprising oxaliplatin at a dose of 85 mg/m² oxaliplatin by intravenous infusion;
c. followed by administering to said patient a pharmaceutical composition comprising a pharmaceutically acceptable salt of folinic acid (LV) at a dose of 400 mg/m² folinic acid by intravenous infusion;
d. followed by administering to said patient a pharmaceutical composition comprising 5-fluorouracil (5-FU) at a dose of 400 mg/m² 5-FU by intravenous bolus; and
e. followed by administering a pharmaceutical composition comprising 5-fluorouracil (5-FU) at a dose of 2,400 mg/m² 5-FU by intravenous infusion over 46 hours.

In one embodiment, the methods of treating colorectal cancer and metastatic colorectal cancer, the methods produce no progression of said solid tumors after 2, 4, 8, 16, or 24 weeks of treatment after repeating steps (a) to (f) about every two weeks. In another embodiment, the methods of treating colorectal cancer and metastatic colorectal cancer produce no statistically significant progression of said solid tumors after 2, 4, 8, 16, or 24 weeks of treatment after repeating steps (a) to (f) about every two weeks.

In yet another embodiment, the methods of treating colorectal cancer and metastatic colorectal cancer, the methods produce no progression of said solid tumors after 2, 4, 8, 16, or 24 weeks of treatment after repeating steps (a) to (f) about every two weeks compared to treatment of solid tumors in a patient diagnosed with colorectal cancer or metastatic colorectal cancer treated by administering at least bevacizumab, oxaliplatin, 5-FU and folinic acid (LV), wherein the administering of at least bevacizumab, oxaliplatin, 5-FU and LV comprises the steps of:
a. administering to said patient a pharmaceutical composition comprising bevacizumab at a dose of 5 mg/kg bevacizumab by intravenous infusion;
b. followed by administering to said patient a pharmaceutical composition comprising oxaliplatin at a dose of 85 mg/m² oxaliplatin by intravenous infusion;
c. followed by administering to said patient a pharmaceutical composition comprising a pharmaceutically acceptable salt of folinic acid (LV) at a dose of 400 mg/m² folinic acid by intravenous infusion;
d. followed by administering to said patient a pharmaceutical composition comprising 5-fluorouracil (5-FU) at a dose of 400 mg/m² 5-FU by intravenous bolus; and
e. followed by administering a pharmaceutical composition comprising 5-fluorouracil (5-FU) at a dose of 2,400 mg/m² 5-FU by intravenous infusion over 46 hours.

In one embodiment, the methods disclosed herein of treating a patient diagnosed with colorectal cancer or metastatic colorectal cancer is a first-, second-, third-, fourth- or fifthline treatment.

In one embodiment, the methods disclosed herein of treating a patient diagnosed with colorectal cancer or metastatic colorectal cancer has an overall response rate (ORR) of at least 40, 50, 60, 70 or 75% after 8 weeks. In another embodiment, the method has an overall response rate (ORR) of at least 40, 50, 60, 70 or 75% after 16 weeks. In yet another embodiment, the methods disclosed herein maintain stable disease between 8 and 16 weeks of treatment.

In another embodiment, the methods disclosed herein of treating a patient diagnosed with colorectal cancer or metastatic colorectal cancer has an overall response rate (ORR) of at least 10, 15, 20, 25, 30 or 35% greater than a method of treating patients diagnosed with colorectal cancer or metastatic colorectal cancer for 8 or 16 weeks comprising the steps of:
a. administering to said patient a pharmaceutical composition comprising
   bevacizumab at a dose of 5 mg/kg bevacizumab by intravenous infusion;
b. followed by administering to said patient a pharmaceutical composition
   comprising oxaliplatin at a dose of 85 mg/m2 oxaliplatin by intravenous infusion;
c. followed by administering to said patient a pharmaceutical composition comprising a pharmaceutically acceptable salt of folinic acid (LV) at a dose of 400 mg/m2 folinic acid by intravenous infusion;
d. followed by administering to said patient a pharmaceutical composition comprising 5-fluorouracil (5-FU) at a dose of 400 mg/m2 5-FU by intravenous bolus; and
e. followed by administering a pharmaceutical composition comprising 5-fluorouracil (5-FU) at a dose of 2,400 mg/m2 5-FU by intravenous infusion over 46 hours.

In one embodiment, the patient diagnosed with colorectal cancer or metastatic colorectal cancer has not received any prior cancer treatment, including but not limited to chemotherapy, radiotherapy, immunotherapy, or surgery. In another embodiment, the patient diagnosed with colorectal cancer or metastatic colorectal cancer has not received any prior cancer treatment, except chemotherapy or radiotherapy for colorectal cancer shorter than 8 weeks duration, including but not limited to chemotherapy, radiotherapy, immunotherapy, or surgery less than 6 months before initiation of the treatment methods disclosed herein.

In one embodiment where the patient diagnosed with colorectal cancer or metastatic colorectal cancer has not received any prior cancer treatment, the methods disclosed herein produce a partial response after 8 or 16 weeks of treatment. In another embodiment where the patient diagnosed with colorectal cancer or metastatic colorectal cancer has not received any prior cancer treatment, the methods disclosed herein produce stable disease after 8 or 16 weeks of treatment. In yet another embodiment, where the patient diagnosed with colorectal cancer or metastatic colorectal cancer has not received any prior cancer treatment, the methods disclosed herein produce an overall response rate (ORR) of at least 50% after 8 weeks. In a further embodiment where the patient diagnosed with colorectal cancer or metastatic colorectal cancer has not received any prior cancer treatment, the methods of the present invention produces an ORR of at least 60% after 8 weeks. In a yet a further embodiment where the patient diagnosed with colorectal cancer or metastatic colorectal cancer has not received any prior cancer treatment, the methods of the present invention produces an ORR of at least 70% after 8 weeks.

In one embodiment, where the patient diagnosed with colorectal cancer or metastatic colorectal cancer has not received any prior cancer treatment, the methods disclosed herein produce an ORR of at least 20% greater than a method of treating patients diagnosed with colorectal cancer or metastatic colorectal cancer for 8 or 16 weeks comprising the steps of:
a. administering to said patient a pharmaceutical composition comprising bevacizumab at a dose of 5 mg/kg bevacizumab by intravenous infusion;
b. followed by administering to said patient a pharmaceutical composition comprising oxaliplatin at a dose of 85 mg/m2 oxaliplatin by intravenous infusion;
c. followed by administering to said patient a pharmaceutical composition comprising a pharmaceutically acceptable salt of folinic acid (LV) at a dose of 400 mg/m2 folinic acid by intravenous infusion;
d. followed by administering to said patient a pharmaceutical composition comprising 5-fluorouracil (5-FU) at a dose of 400 mg/m2 5-FU by intravenous bolus; and
e. followed by administering a pharmaceutical composition comprising 5-fluorouracil (5-FU) at a dose of 2,400 mg/m2 5-FU by intravenous infusion over 46 hours.

In another embodiment, where the patient diagnosed with colorectal cancer or metastatic colorectal cancer has not received any prior cancer treatment, the methods disclosed herein produce an ORR of at least 25% greater than a method of treating patients diagnosed with colorectal cancer or metastatic colorectal cancer for 8 or 16 weeks comprising the steps of:
a. administering to said patient a pharmaceutical composition comprising bevacizumab at a dose of 5 mg/kg bevacizumab by intravenous infusion;
b. followed by administering to said patient a pharmaceutical composition comprising oxaliplatin at a dose of 85 mg/m2 oxaliplatin by intravenous infusion;
c. followed by administering to said patient a pharmaceutical composition comprising a pharmaceutically acceptable salt of folinic acid (LV) at a dose of 400 mg/m2 folinic acid by intravenous infusion;
d. followed by administering to said patient a pharmaceutical composition comprising 5-fluorouracil (5-FU) at a dose of 400 mg/m2 5-FU by intravenous bolus; and
e. followed by administering a pharmaceutical composition comprising 5-fluorouracil (5-FU) at a dose of 2,400 mg/m2 5-FU by intravenous infusion over 46 hours.

In yet another embodiment, where the patient diagnosed with colorectal cancer or metastatic colorectal cancer has not received any prior cancer treatment, the methods disclosed herein produce an ORR of at least 30% greater than a method of treating patients diagnosed with colorectal cancer or metastatic colorectal cancer for 8 or 16 weeks comprising the steps of:
a. administering to said patient a pharmaceutical composition comprising bevacizumab at a dose of 5 mg/kg bevacizumab by intravenous infusion;
b. followed by administering to said patient a pharmaceutical composition comprising oxaliplatin at a dose of 85 mg/m2 oxaliplatin by intravenous infusion;
c. followed by administering to said patient a pharmaceutical composition comprising a pharmaceutically acceptable salt of folinic acid (LV) at a dose of 400 mg/m2 folinic acid by intravenous infusion;
d. followed by administering to said patient a pharmaceutical composition comprising 5-fluorouracil (5-FU) at a dose of 400 mg/m2 5-FU by intravenous bolus; and
e. followed by administering a pharmaceutical composition comprising 5-fluorouracil (5-FU) at a dose of 2,400 mg/m2 5-FU by intravenous infusion over 46 hours.

In a further embodiment, where the patient diagnosed with colorectal cancer or metastatic colorectal cancer has not received any prior cancer treatment, the methods disclosed herein produce an ORR of at least 35% greater than a method of treating patients diagnosed with colorectal cancer or metastatic colorectal cancer for 8 or 16 weeks comprising the steps of:
a. administering to said patient a pharmaceutical composition comprising bevacizumab at a dose of 5 mg/kg bevacizumab by intravenous infusion;
b. followed by administering to said patient a pharmaceutical composition comprising oxaliplatin at a dose of 85 mg/m2 oxaliplatin by intravenous infusion;
c. followed by administering to said patient a pharmaceutical composition comprising a pharmaceutically acceptable salt of folinic acid (LV) at a dose of 400 mg/m2 folinic acid by intravenous infusion;
d. followed by administering to said patient a pharmaceutical composition comprising 5-fluorouracil (5-FU) at a dose of 400 mg/m2 5-FU by intravenous bolus; and
e. followed by administering a pharmaceutical composition comprising 5-fluorouracil (5-FU) at a dose of 2,400 mg/m2 5-FU by intravenous infusion over 46 hours.

### DETAILED DESCRIPTION OF THE INVENTION

### 1. Colorectal Cancer

Colorectal cancer (CRC) and metastatic colorectal cancers (mCRC) are the third most common cancers in men (10% of the total) and the second in women (9.2%), with over 1.3 Million cases (746 000 men and 614 000 women) reported worldwide during 2012. The geographic incidence of CRC varies widely across the world, and the geographical patterns are very similar in men and women. Incidence rates vary ten-fold in both sexes worldwide, the highest estimated rates being in Australia/New Zealand (ASR 44.8 and 32.2 per 100,000 in men and women respectively), and the lowest in Western Africa (4.5 and 3.8 per 100,000). The incidence increases with age and is highest amongst the elder population, i.e. 60-64 years: 67.4; 65-69 years: 95.1; 70-74 years: 127.8; and ≥ 75 years: 196.2 per 100 000 [Ferlay J, Soerjomataram I, Ervik M, Dikshit R, Eser S, Mathers C, Rebelo M, Parkin DM, Forman D, Bray, F. GLOBOCAN 2012 v1.0, Cancer Incidence and Mortality Worldwide: IARC CancerBase No. 11. Lyon, France: International Agency for Research on Cancer; 2013].

Approximately 40-50% of the affected patients develop metastatic disease and more than half a million deaths are reported annually as a consequence of CRC [Jemal A, Bray F, Center MM, Ferlay J, Ward E, Forman D. Global cancer statistics. CA Cancer J Clin. 2011 Mar-Apr;61(2):69-90]. Indeed CRC accounted for 694 000 deaths worldwide solely during 2012 (8.5% of the total) [Ferlay J, Soerjomataram I, Ervik M, Dikshit R, Eser S, Mathers C, Rebelo M, Parkin DM, Forman D, Bray, F. GLOBOCAN 2012 v1.0, Cancer Incidence and Mortality Worldwide: IARC CancerBase No. 11 [Internet]. Lyon, France: International Agency for Research on Cancer; 2013].

CRC patients are usually treated surgically and, in most circumstances, with curative intent. Surgery, in fact, remains the primary modality of treatment for malignancies of the lower gastrointestinal tract, and standard resection is the only therapy required for early-stage cancer [Nelson H, Petrelli N, Carlin A, Couture J, Fleshman J, Guillem J, et al. Guidelines 2000 for colon and rectal cancer surgery. J Natl Cancer Inst. 2001 Apr 18;93(8):583-96]. As the stage of the tumour advances, in terms of depth of penetration and lymph node involvement, the chance of cure with surgery alone diminishes and the rate of local recurrence increase. In such cases, surgery may either be combined with adjuvant treatment or be performed for palliative control of symptoms only.

In one embodiment, the methods of treating cancer disclosed herein are practiced on a patient diagnosed with cancer. In a preferred embodiment the patient diagnosed with cancer has at least one solid tumor. In another embodiment of the disclosed methods, the patient is diagnosed with a cancer selected from the group consisting of colon cancer, stomach cancer, breast cancer, bowel cancer, gallbladder cancer, lung cancer, colorectal cancer, metastatic CRC, head and neck cancer, liver cancer, pancreatic cancer and osteosarcoma.

The methods of treating colorectal cancer and metastatic colorectal cancer disclosed herein are practiced on a patient diagnosed with colorectal cancer or metastatic colorectal cancer having at least one solid tumor. In another embodiment, the colorectal cancer is colorectal adenocarcinoma and in yet another embodiment the colorectal cancer or colorectal adenocarcinoma is verified by biopsy.

### 2. Chemotoxic Agents

In separate embodiments the disclosed methods of treating a patient diagnosed with cancer comprises administration of one or more drugs selected from: Platinum Drugs, such as cisplatin (CDDP), carboplatin (CBDCA) and oxaliplatin (oloxetin); Antimetabolites, such as 5-fluoruracil (5-FU), capecetabine (Xeloda), gemcitabine(Gemzar ), methotrexate and pemetrexed (Alimta); Anti-tumor antibiotics, such as doxorubicin (Adriamycin), daunorubicin, actinomycin-D and mitomycin-C (MTC); Topoisomerase Inhibitors, such as irinotecan (CPT-11), topotecan (hycamtin) and etoposide (VP-16); Mitotic Inhibitors, such as paclitaxel (Taxol), docetaxel (Taxotere) and vincristine (Oncovin); Corticosteroids, such as prednisone, methylprednisolone (Solumedrol) and dexamethasone (Decadron); Monoclonal Antibodies (MABs), such as cetuximab (Erbitux), rituximab (Rituxan) and bevacizumab (Avastin); Small Molecular EGFR Inhibitors, such as gefitinib (Iressa); Hormone Therapies, such as tamoxifen (Nolvadex) and bicalutamide (Casodex), Immune Check Point Inhibitors, such as PD-1 inhibitors such as pembrolizumab (Keytruda) and nivolumab (Opdivo); PD-L1 Inhibitors, such as atezolizumab (Tecentriq); and Cancer Vaccines.

In one embodiment, the disclosed methods of treating a patient diagnosed with cancer comprises administration of a fluorinated pyrimidine base, such as 5-fluorouracil (5-FU). In one embodiment, the fluorinated pyrimidine base is 2'-deoxy-5 fluorouridine or 5'-deoxy-5-fluorouridine.

5-FU is formulated as injection solution. 5-FU may be administered as an IV bolus on day 1 and continuous IV infusion over 46 hours in each treatment cycle. 5-FU may be administered approximately 60 minutes after start of oxaliplatin administration, respectively. The treatment may be repeated every two weeks. An administered 5-FU IV bolus dose should not surpass the maximum recommended daily dose of 1000 mg, regardless of the body surface area.

In one embodiment, the disclosed methods of treating a patient diagnosed with cancer comprises administration of an alkylating antineoplastic agent. In another embodiment, the alkylating antineoplastic agent is contains a platinum (II) center. In another embodiment, the alkylating antineoplastic agent is cisplatin (CDDP), carboplatin (CBDCA) or oxaliplatin (oloxetin). In a preferred embodiment, the alkylating antineoplastic agent is oxaliplatin.

Oxaliplatin is formulated as a concentrated infusion solution. Oxaliplatin may be administered as IV infusion over 15 - 120 minutes on Day 1 in each treatment cycle and repeated every two weeks. Caution will be taken regarding toxicity associated with administration that may affect rate of infusion (e.g. grade ≤2 allergy, laryngopharyngeal dysesthesias, and laryngeal spasm). In such cases, the rate of Oxaliplatin administration should be prolonged in subsequent treatment cycles according to clinical practice recommendations.

In one embodiment, the disclosed methods of treating a patient diagnosed with cancer comprises administration of a recombinant humanized monoclonal antibody directed against the vascular endothelial growth factor (VEGF). In another embodiment the disclosed methods of treating a patient diagnosed with cancer comprises administration of bevacizumab.

In one embodiment, the recombinant humanized monoclonal antibody directed against VEGF is selected from the group consisting of anti-VEGF humanized monoclonal antibody, anti-VEGF monoclonal antibody, anti-VEGF rhuMAb, bevacizumab biosimilar BAT1706, bevacizumab biosimilar BEVZ92, bevacizumab biosimilar BI 695502, bevacizumab biosimilar CBT 124, bevacizumab biosimilar FKB238, bevacizumab, biosimilar HD204, bevacizumab biosimilar HLX04, bevacizumab biosimilar MB02, bevacizumab biosimilar MIL60, bevacizumab biosimilar PF-06439535, bevacizumab biosimilar QL 1101, immunoglobulin G1 (human-mouse monoclonal rhuMab-VEGF gamma-chain anti-human vascular endothelial growth factor), disulfide with human-mouse monoclonal rhuMab-VEGF light chain, and dimer recombinant humanized anti-VEGF monoclonal antibody.

Bevacizumab is formulated as a concentrated infusion solution. Bevacizumab may be administered as an intravenous infusion as 5 mg/kg of body weight given once every 2 weeks. It will be administered on Day 1 of every 2-week cycle and repeated every two weeks. The initial dose should be delivered over 90 minutes as an intravenous infusion. If the first infusion is well tolerated, the second infusion may be administered over 60 minutes. If the 60-minute infusion is well tolerated, all subsequent infusions may be administered over 30 minutes. It should not be administered as an intravenous push or bolus.

In one embodiment, the disclosed methods of treating a patient diagnosed with cancer comprises administration of a recombinant fusion protein consisting of VEGF-binding portions from the extracellular domains of human VEGF Receptors 1 and 2 fused to the Fc portion of the human IgG1, including but not limited to Ziv-aflibercept.

In one embodiment, the disclosed methods of treating a patient diagnosed with cancer comprises administration of a recombinant human IgG1 monoclonal antibody that specifically binds to vascular endothelial growth factor receptor 2, including but not limited to ramucirumab.

### 3. 6R-MTHF and LV

In one embodiment, [6R]-5,10-methylenetetrahydrofolic acid (6R-MTHF) or a pharmaceutically acceptable salt thereof is employed as a solid form which is soluble in water or as a lyophilisate, optionally stabilized by one or more suitable excipients and/or antioxidants such as citric acid or ascorbic acid or salt forms thereof.

In one embodiment, 6R-MTHF may be in the form of a free acid, in the form of pharmaceutically acceptable salts, particularly acidic salts, as well as alkali or alkaline earth metal salts. Examples of pharmaceutically acceptable salts include, but are not limited to, mineral or organic acid salts of basic residues such as amines; alkali or organic salts of acidic residues such as carboxylic acids; and the like. In one embodiment, the pharmaceutically acceptable salt of 6R-MTHF is the hemisulfate salt. In certain embodiments, the pharmaceutically acceptable salts of 6R-MTHF used in the methods disclosed herein may be provided as a lyophilisate. In another embodiment, the lyophilisates used in the methods disclosed herein are prepared from 6R-MTHF hemisulfate salt. In a preferred embodiment, the lyophilisate used in the methods disclosed herein is prepared from a 6R-MTHF hemisulfate salt and further comprises other salts and excipients and in one embodiment trisodium citrate dihydrate.

In certain embodiments, the lyophilisates used in the methods disclosed herein are reconstituted in aqueous media prior to use. In one embodiment, the aqueous media is water or saline. In a preferred embodiment, the aqueous media is water. In other embodiments, the lyophilisate comprises 100 mg of 6-MTHF. In a preferred embodiment, the lyophilisate comprising 100 mg 6R-MTHF is reconstituted by the addition of 10 mL water. In yet another embodiment, the lyophilisate comprising 100 mg of 6R-MTHF is provided in a container. In another embodiment of the methods disclosed herein, the reconstituted lyophilisate forms a solution with a concentration of between 5 and 20 mg/mL 6R-MTHF. In a preferred embodiment, the reconstituted lyophilisate forms a solution with a concentration of between 7 and 12 mg/mL 6R-MTHF. More preferably, the reconstituted lyophilisate forms a solution with a concentration of 10 mg/mL 6R-MTHF. In other embodiments of the methods disclosed herein, the reconstituted lyophilisate is isotonic. In yet another embodiment, the reconstituted lyophilisate has a pH of between 7.0 and 9.0, more preferably between pH 8.0 and 9.0, most preferably between 8.3 and 8.7 and even more preferably pH 8.5.

The 6R-MTHF used in the methods disclosed herein is diastereoisomerically pure, natural 6R-MTHF. As used herein, the term "diastereoisomerically pure" means 6R-MTHF or its salt in isomeric excess over the other isomer with a diastereomeric excess (d.e.) of at least 98% d.e., preferably 99% d.e., more preferably 99.8% d.e. and most preferably 99.9% d.e.

In another embodiment, the 6R-MTHF is chemically pure. As used herein, the term "chemically pure" means a compound in about 80% chemical purity, preferably about 90%, more preferably about 95%, more preferably about 97%, more preferably about 98% chemical purity, and most preferably 99% or higher than 99%, e.g., 99.5, 99.6, 99.7, 99.8, 99.9 or up to 100% chemical purity, as determined by HPLC. Chemical impurities may include unreacted starting material (including solvents), degradation products of 6R-MTHF (such as tetrahydrofolic acid and its degradation products), etc.

Leucovorin (LV) is provided as calcium folinate in a 20 mL solution at a concentration of 10 mg/ml which is available from a numer of commercial sources. The LV solution may contain any number of excipients, including but not limited to sodium chloride.

### 4. Overall Response Rate, Progressive and Stable Disease

As used herein, "Overall Response Rate (ORR)" means the proportion of patients with reduction in tumor burden of a predefined amount.

The ORR may be calculated as follows: ORR = sum of partial responses plus complete responses as per RECIST 1.1 (a set of published rules that define when tumours in cancer patients progress during treatments)( [Eisenhauer EA, Therasse P, Bogaerts J, Schwartz LH, Sargent D, Ford R, et al. New response evaluation criteria in solid tumours: revised RECIST guideline (version 1.1). Eur J Cancer. 2009 Jan; 45(2):228-47; incorporated by reference in its entirety]. As defined herein, a "complete response (CR)" means the disappearance of all target lesions. Any pathological lymph nodes (whether target or non-target) must have reduction in short axis to <10 mm. As used herein, a "partial response (PR)" means at least a 30% decrease in the sum of diameters of target lesions, taking as reference the baseline sum diameters.As used herein, "progressive disease (PD)" means at least a 20% increase in the sum of diameters of target lesions, taking as reference the smallest sum on study (this includes the baseline sum if that is the smallest on study). In addition to the relative increase of 20%, the sum must also demonstrate an absolute increase of at least 5 mm. The appearance of one or more new lesions is also considered progression.

As used herein, "stable disease (SD)" means neither sufficient shrinkage to qualify for PR nor sufficient increase to qualify for PD, taking as reference the smallest sum diameters while on study.

As used herein, "progression free" refers to a patient remaining alive, without the cancer progressing or getting worse. Cancer or tumor progression is based on CT/MRI scans according to RECIST 1.1 definitions (incorporated herein by reference)[Eisenhauer et al. (2009) New response evaluation criteria in solid tumours: Revised RECIST guideline (version 1.1) 45: 228-247].

As used herein, "progression-free survival (PFS)" means the time from randomization in a clinical trial to tumor progression or death, whichever comes first. In one embodiment of the present invention, PFS is measured after 1, 2, 3, 4 or 5 years. In a preferred embodiment, PFS is measured after 3 years.

As used herein, "overall survival (OS)" is defined as the time from randomization in a clinical trial to the time of death from any cause. In one embodiment of the present invention, OS is measured after 3 or 5 years. In a preferred embodiment, PFS is measured after 5 years.

As used herein, "first line" therapy is the chemotherapy used for a patient who has not previously been treated for cancer in a pre-defined stage (such as Stage I, Stage II, Stage III or Stage IV). As used herein, "second line" treatment means the therapy received from the time that the first-line chemotherapy backbone has to be changed, mostly after failure of a first-line strategy. Later-line therapy (such as second-, third-, fourth line, etc.) is the chemotherapy applied after failure of earlier-line strategies.

CT scans or MRIs will be performed of the thorax, abdomen, and pelvis at baseline and every 8 weeks after randomization. As used herein, "baseline scan" refers to the CT scan or MRI obtained within 28 days of initiating treatment using the methods disclosed herein. As used herein, "baseline size" refers to the size of a solid tumor as determined by a baseline scan.

### EXAMPLES

The following examples are merely indicative of the nature of the present invention, and should not be construed as limiting the scope of the invention, nor of the appended claims, in any manner.

### EXAMPLE 1--Clinical Study Design

A multicenter, randomized, parallel-group, Phase IIb/III study in approximately 440 patients with advanced colorectal cancer is conducted to compare the efficacy of treatment with 6R-MTHF versus LV in combination with 5-FU, oxaliplatin, and bevacizumab.

This is a randomized, multicenter, parallel-group, Phase IIb/III study to compare the efficacy of arfolitixorin versus LV in patients with advanced CRC treated by 5-FU, oxaliplatin, and bevacizumab.

Patients will be randomized in a 1:1 ratio to either the investigational arm (arfolitixorin) or the comparator arm (Leucovorin), following the completion of all screening assessments and after confirmation of their eligibility, using a stratified permuted block randomization. Randomization will be stratified for the following baseline factors:
- Geographic region (Europe / North America)
- Primary tumor location (left colon/right colon/rectal cancer)
- Previous adjuvant CRC treatment

All patients will be treated according to the study protocol. Tumor evaluations, based on CT/MRI scans performed every 8 weeks, will be performed by blinded centrally adjudicated assessments according to RECIST 1.1 definitions (incorporated herein by reference) [Eisenhauer et al. (2009) New response evaluation criteria in solid tumours: Revised RECIST guideline (version 1.1) 45: 228-247].

CT scans or MRIs will be performed of the thorax, abdomen, and pelvis at screening (baseline) and every 8 weeks after randomization until PD and at the end of treatment visit. A radiologist at each study site/hospital (preferably the same radiologist for all patients at that hospital) is to evaluate each scan for target lesions. The following information is to be collected:
- Patient ID
- Date of procedure
- Type of procedure
- Number and size of measurable site(s) of disease according to RECIST 1.1 criteria.

To minimize bias and have an objective assessment of the tumor response, a central imaging vendor will perform independent and blinded response evaluations retrospectively.

The central reading process will be done from screening until progressive disease (PD) by two different Central Readers (double review with adjudication). All the images will be provided to the Central Readers in an organized chronological sequence. In order to remove the bias of interpolation and ensure the reader concentrates on the latest time-point images and has no idea that there are more to follow, each time-point images will be displayed, scored and locked in the database before the assessment of the following time point.

The methodology applied at baseline regarding the identification of the target and non-target lesions is based on a common lesions selection process and an identical usage of those selected lesions by both Central Readers.

The rationale for this lesion selection process at baseline is based on the fact that readers can potentially identify different target and non-target lesions that each considers representative of the subject's overall extent of disease. There is also the potential for the Central Readers to identify the same lesions but classify them differently between target and non-target lesions. Discordance in lesion selection can reach 40% or even more. In addition, since lesions do not respond or progress at the same rate, differences between readers with regard to response or progression of lesions can be observed in the subsequent time-points.

To minimize such important risk of discordance, the following process for the initial CT-scan or MRI will be applied.

One of the two primary Central Readers is randomly selected to perform lesions selection on initial images:
- Determines the number of target lesions and non-target lesions based on RECIST 1.1,
- Communicates the target and not target lesions identification to the second primary Central Reader, via the central imaging vendor to preserve the independence of the two Central Readers.
- Tracks lesions locations and labelling for up to 5 (2 per organ) target lesions and 10 non-target lesions on the subsequent time points.

After having defined the type of targets and others lesions to be selected, the two primary Central Readers will carry out a separate and blinded measurement of the target lesions at Baseline:
- If there is an agreement between Central Readers (Δ ∑ <20% and Δ <15% for each lesion), Central Reader will keep his own ∑ for the next time points,

In case of discrepancies between Central Readers (Δ ∑ >20% or Δ >15% for any lesion), the Central Chair/adjudicator) will adjudicate the result of the two previous Central Readers and will provide the reference values for the baseline (for each target lesion). This value will be considered as reference by both Central Readers for the next time point analyses.

Tumor imaging at screening must be performed within 28 days prior to the date of randomization. Scans performed as part of routine clinical management are acceptable for use as screening tumor imaging if they are of diagnostic quality and performed within 28 days prior to the date of randomization and can be assessed by the central imaging vendor.

Eligible and consenting patients will be randomized 1:1 to one of two treatment groups (A and B) and administered one of the following treatment regimens starting on the first day of each 14-day treatment cycle:

Group A (ARFOX + bevacizumab):
- Bevacizumab 5 mg/kg, IV infusion in accordance with the label ↓followed by
- Oxaliplatin 85 mg/m2 IV infusion in accordance with the label ↓followed by
- 5-FU rapid IV bolus 400 mg/m2 ↓followed by (30 minutes after 5-FU bolus)
- Arfolitixorin (6R-MTHF) 60 mg/m2 mg/m2 rapid IV bolus (less than 3 minutes) ↓followed by
- 5-FU 2400 mg/m2 continuous IV infusion over 46 hours ↓followed by (30 minutes after start of 5-FU IV infusion)
- Arfolitixorin (6R-MTHF) 60 mg/m2 mg/m2 rapid IV bolus (less than 3 minutes)

Group B (modified FOLFOX-6 + bevacizumab):
- Bevacizumab 5 mg/kg, IV infusion in accordance with the label ↓followed by
- Oxaliplatin 85 mg/m2 IV infusion in accordance with the label ↓concurrently with
- LV 400 mg/m2 IV infusion in accordance with the label ↓followed by
- 5-FU rapid IV bolus 400 mg/m2 ↓followed by (30 minutes after 5-FU bolus)
- 5-FU 2400 mg/m2 continuous IV infusion over 46 hours

The doses of 6R-MTHF and LV should not be adjusted. Following a treatmentrelated adverse event the doses of 5-FU, oxaliplatin, and bevacizumab may be adjusted or delayed in accordance with labelling. Oxaliplatin cannot be substituted but can be removed from the treatment regimens after the induction phase of treatment (8 treatment cycles) at the Investigator's discretion. For patients with platinum hypersensitivity, pre-medication with IV dexamethasone, chlorphenamine, and ranitidine can be given at the Investigator's discretion.

For inclusion in the study, patients must fulfill all the following criteria:
1. Colorectal adenocarcinoma verified by biopsy.
2. Availability of biopsy material, from the primary tumor or metastasis, allowing for analysis of tumor gene expression.
3. Non-resectable metastatic CRC eligible for first line therapy with 5-FU, oxaliplatin, and bevacizumab.
4. Evaluable disease with at least one measurable lesion of metastatic disease according to RECIST 1.1 criteria (>10 mm in longest diameter on axial image on CT-scan or MRI with <5 mm reconstruction interval) obtained within 28 days of randomization.
5. Life expectancy of more than 4 months.
6. Eastern Cooperative Oncology Group (ECOG)/World Health Organization (WHO) Performance Status Scale performance status 0 or 1.
7. Hemoglobin (Hb) > 100 g/L, Absolute neutrophil count (ANC) > 1.5 x 109 /L, Thrombocytes > 100 x 109 /L.
8. Creatinine clearance > 50 mL/min, Total bilirubin < 1.5 x ULN, AST and ALT < 3 x ULN (and < 5 x ULN in case of liver metastases).
9. Male or female ≥18 years of age.
10. Female patients of childbearing potential must have a negative urine pregnancy test and use adequate contraceptive measures. Male patients must use adequate contraceptive measures.
11. Voluntarily signed informed consent prior to any study related procedure not part of normal medical care, with the understanding that consent may be withdrawn by the patient at any time without prejudice to future medical care.

Patients meeting one or more of the following criteria are ineligible to participate in the study:
1. Malignant tumors other than colorectal adenocarcinomas (current or within the previous five years), with the exception for curatively treated nonmelanoma skin cancer or in situ carcinoma of the cervix.
2. Less than 6 months between randomization and the last anticancer treatment administration (chemotherapy/ radiotherapy/immunotherapy/surgery, etc.). (NB: Rectal cancer treatment shorter than 8 weeks of chemo/radiation therapy is allowed.)
3. Signs of disease progression during the 6 months after prior anticancer before randomization.
4. Indication for any mCRC surgery or anti-cancer treatment other than study treatment.
5. Prior treatment with arfolitixorin.
6. Indication for treatment with a nucleoside analogue (e.g., sorivudine), a dihydropyrimidine dehydrogenase (DPD) inhibitor (e.g., brivudin), a 5-FU analogue, or 5-FU for a condition other than mCRC.
7. Known DPD deficiency.
8. Known or suspected central nervous system metastases.
9. Unresolved bowel obstruction, uncontrolled Crohn's disease, or ulcerative colitis.
10. History of cardiac disease with a New York Heart Association Class II or greater, congestive heart failure, myocardial infarction, or unstable angina at any time during the 6 months prior to randomization, or serious arrhythmias requiring medication for treatment.
11. Current severe chronic diarrhea.
12. Current chronic infection or uncontrolled serious illness causing immunodeficiency.
13. Known or suspected hypersensitivity or intolerance to arfolitixorin, LV, 5-FU, oxaliplatin, or bevacizumab.
14. Breastfeeding patients.
15. Patient who received investigational drugs in other clinical trials within 28 days, or 5 half-lives of the investigational drug, prior to randomization.
16. Patient with serious medical or psychiatric illness likely to interfere with participation in this clinical study.
17. Ongoing drug or alcohol abuse.
18. Any condition that, in the opinion of the Investigator, could compromise the patient's safety or adherence to the study protocol.
19. Involvement, or related to people involved in the planning or conduct of the study (applies to both Isofol Medical AB staff and staff at the study site).

Overall Response Rate (ORR) will be analyzed using a Cochran-Mantel-Haenszel test (CMH), stratified for the stratification factors used for randomization (geographic region, primary tumor location and previous adjuvant CRC treatment).

### EXAMPLE 2--Treatment of Patient Diagnosed with Colorectal Cancer

An 81-year old female (71 kg; 155 cm) was diagnosed with stage IV metastatic colorectal cancer by PET/CT scan. Colorectal adenocarcinoma was verified by biopsy obtained by sigmoidectomy. Three solid tumors (baseline (prior to treatment) CT measurements: 25, 13 and 12 mm) were identified in the abdomen. The patient had previously been treated for colorectal cancer with eloxatin, which had been discontinued prior to the treatment disclosed here.

The patient received treatment comprising the administration of bevacizumab, oxaliplatin, 5-FU and 6R-MTHF as a second-line therapy. Bevacizumab was administered at a dose of 5 mg/kg by intravenous infusion over 30 to 90 minutes. Following administration of bevacizumab, oxaliplatin was administered at a dose of 85 mg/m² by intravenous infusion between 15 and 120 minutes. Following oxaliplatin administration, 5-fluorouracil (5-FU) at a dose of 400 mg/m² was administered by intravenous bolus within 5 minutes. Thirty minutes after administration of the 5-FU intravenous bolus, 6R-MTHF at a dose of 60 mg/m² was administered by intravenous bolus (completed within 3 minutes). About 5 minutes after completion of the 6R-MTHF intravenous bolus administration, an intravenous infusion of 5-FU at a dose of 2,400 mg/m² was initiated. The infusion was paused to administer an intravenous bolus of 6R-MTHF at a dose of 60 mg/m² (completed within 3 minutes). The 5-FU infusion was maintained for 46 hours.

The treatment cycle was repeated once every two weeks for four cycles (8 weeks). During the second cycle, the oxaliplatin dose was reduced to 65 mg/m² and the 5-FU intravenous infusion dose was reduced to 1,800 mg/m². After 8 weeks of treatment, the 25, 13 and 12 mm lesions were unchanged in size (CT measurement made at 10 weeks plus 2 days). The treatment produced stable disease over the 8-week treatment period.

### EXAMPLE 3--Treatment of Patient Diagnosed with Colorectal Cancer

A 48-year old female (67 kg; 160 cm) was diagnosed with stage IV metastatic colorectal cancer by surgery. Colorectal adenocarcinoma was verified by biopsy obtained by masectomy. Two solid tumors (baseline (prior to treatment) CT measurements: 10 and 16 mm) were identified in the liver and lung. The patient had previously been treated for colorectal cancer with bevacizumab, which had been discontinued prior to the treatment disclosed here.

The patient received treatment comprising the administration of bevacizumab, oxaliplatin, 5-FU and 6R-MTHF as a second-line therapy. Bevacizumab was administered at a dose of 5 mg/kg by intravenous infusion over 30 to 90 minutes. Following administration of bevacizumab, oxaliplatin was administered at a dose of 85 mg/m² by intravenous infusion between 15 and 120 minutes. Following oxaliplatin administration, 5-fluorouracil (5-FU) at a dose of 400 mg/m² was administered by intravenous bolus within 5 minutes. Thirty minutes after administration of the 5-FU intravenous bolus, 6R-MTHF at a dose of 60 mg/m² was administered by intravenous bolus (completed within 3 minutes). About 5 minutes after completion of the 6R-MTHF intravenous bolus administration, an intravenous infusion of 5-FU at a dose of 2,400 mg/m² was initiated. The infusion was paused to administer an intravenous bolus of 6R-MTHF at a dose of 60 mg/m² (completed within 3 minutes). The 5-FU infusion was maintained for 46 hours.

The treatment cycle was repeated once every two weeks for four cycles (8 weeks). Treatment led to the disappearance of the 10 mm lesion. The 16 mm lesion was reduced to CT-verified 13 mm. The treatment produced a partial response and no progression of the solid tumors after 8 weeks of treatment.

The treatment cycle was repeated once every two weeks for an additional eight cycles (16 weeks). The 13 mm lesion was reduced to CT-verified 9 mm after 16 weeks. The 9 mm lesion was unchanged in size between 16 and 24 weeks of treatment. The treatment produced a partial response and no progression of the solid tumors after 16 weeks of treatment and no progression of the solid tumors between 16 and 24 weeks of treatment.

### EXAMPLE 4--Treatment of Patient Diagnosed with Colorectal Cancer

A 73-year old male (105 kg; 165 cm) was diagnosed with stage IV metastatic colorectal cancer by MRI. Colorectal adenocarcinoma was verified by biopsy. One solid tumor (baseline (prior to treatment) CT measurement: 35 mm) was identified in the liver. The patient had previously been treated for colorectal cancer with 5-FU, which had been discontinued prior to the treatment disclosed here.

The patient received treatment comprising the administration of bevacizumab, oxaliplatin, 5-FU and 6R-MTHF as a second-line therapy. Bevacizumab was administered at a dose of 5 mg/kg by intravenous infusion over 30 to 90 minutes. Following administration of bevacizumab, oxaliplatin was administered at a dose of 85 mg/m² by intravenous infusion between 15 and 120 minutes. Following oxaliplatin administration, 5-fluorouracil (5-FU) at a dose of 400 mg/m² was administered by intravenous bolus within 5 minutes. Thirty minutes after administration of the 5-FU intravenous bolus, 6R-MTHF at a dose of 60 mg/m² was administered by intravenous bolus (completed within 3 minutes). About 5 minutes after completion of the 6R-MTHF intravenous bolus administration, an intravenous infusion of 5-FU at a dose of 2,400 mg/m² was initiated. The infusion was paused to administer an intravenous bolus of 6R-MTHF at a dose of 60 mg/m² (completed within 3 minutes). The 5-FU infusion was maintained for 46 hours.

The treatment cycle was repeated once every two weeks for four cycles (8 weeks). After 8 weeks of treatment, the 35 mm lesion was unchanged in size. The treatment produced stable disease over the 8-week treatment period.

The treatment cycle was repeated once every two weeks for an additional eight cycles (16 weeks). The 35 mm lesion was unchanged in size after 16 and 24 weeks. The treatment produced no progression of the solid tumors through 24 weeks of treatment.

### EXAMPLE 5--Treatment of Patient Diagnosed with Colorectal Cancer

A 55-year old male (80 kg; 170 cm) was diagnosed with stage IV metastatic colorectal cancer by biopsy. One solid tumor (baseline (prior to treatment) CT measurement: 80 mm) was identified in the liver.

The patient received second-line treatment comprising the administration of bevacizumab, oxaliplatin, 5-FU and 6R-MTHF as a second-line therapy. Bevacizumab was administered at a dose of 5 mg/kg by intravenous infusion over 30 to 90 minutes. Following administration of bevacizumab, oxaliplatin was administered at a dose of 85 mg/m² by intravenous infusion between 15 and 120 minutes. Following oxaliplatin administration, 5-fluorouracil (5-FU) at a dose of 400 mg/m² was administered by intravenous bolus within 5 minutes. Thirty minutes after administration of the 5-FU intravenous bolus, 6R-MTHF at a dose of 60 mg/m² was administered by intravenous bolus (completed within 3 minutes). About 5 minutes after completion of the 6R-MTHF intravenous bolus administration, an intravenous infusion of 5-FU at a dose of 2,400 mg/m² was initiated. The infusion was paused to administer an intravenous bolus of 6R-MTHF at a dose of 60 mg/m² (completed within 3 minutes). The 5-FU infusion was maintained for 46 hours.

The treatment cycle was repeated once every two weeks for four cycles (8 weeks). After 8 weeks of treatment, the 80 mm lesion increased to 96 mm in size.

### EXAMPLE 6--Treatment of Patient Diagnosed with Colorectal Cancer

A 67-year old male (80 kg; 173 cm) was diagnosed with stage IV metastatic colorectal cancer by PET-CT. Colorectal adenocarcinoma was verified by biopsy. One solid tumor (baseline (prior to treatment) CT measurement: 44 mm) was identified in the liver.

The patient received second-line treatment comprising the administration of bevacizumab, oxaliplatin, 5-FU and 6R-MTHF as a second-line therapy. Bevacizumab was administered at a dose of 5 mg/kg by intravenous infusion over 30 to 90 minutes. Following administration of bevacizumab, oxaliplatin was administered at a dose of 85 mg/m² by intravenous infusion between 15 and 120 minutes. Following oxaliplatin administration, 5-fluorouracil (5-FU) at a dose of 400 mg/m² was administered by intravenous bolus within 5 minutes. Thirty minutes after administration of the 5-FU intravenous bolus, 6R-MTHF at a dose of 60 mg/m² was administered by intravenous bolus (completed within 3 minutes). About 5 minutes after completion of the 6R-MTHF intravenous bolus administration, an intravenous infusion of 5-FU at a dose of 2,400 mg/m² was initiated. The infusion was paused to administer an intravenous bolus of 6R-MTHF at a dose of 60 mg/m² (completed within 3 minutes). The 5-FU infusion was maintained for 46 hours.

The treatment cycle was repeated once every two weeks for four cycles (8 weeks). After 8 weeks of treatment, the 44 mm lesion was reduced in size to 41 mm. The treatment produced stable disease over the 8-week treatment period.

The treatment cycle was repeated once every two weeks for an additional four cycles (8 weeks). The 41 mm lesion was unchanged in size after 16 weeks. The treatment produced stable disease and no progression of the solid tumors through 16 weeks of treatment.

### EXAMPLE 7--Treatment of Patient Diagnosed with Colorectal Cancer

A 56-year old female (56 kg; 163 cm) was diagnosed with stage IV metastatic colorectal cancer by CT. Colorectal adenocarcinoma was verified by biopsy. One solid tumor (baseline (prior to treatment) CT measurement: 22 mm) was identified in the liver.

The patient received second-line treatment comprising the administration of bevacizumab, oxaliplatin, 5-FU and 6R-MTHF as a second-line therapy. Bevacizumab was administered at a dose of 5 mg/kg by intravenous infusion over 30 to 90 minutes. Following administration of bevacizumab, oxaliplatin was administered at a dose of 85 mg/m² by intravenous infusion between 15 and 120 minutes. Following oxaliplatin administration, 5-fluorouracil (5-FU) at a dose of 400 mg/m² was administered by intravenous bolus within 5 minutes. Thirty minutes after administration of the 5-FU intravenous bolus, 6R-MTHF at a dose of 60 mg/m² was administered by intravenous bolus (completed within 3 minutes). About 5 minutes after completion of the 6R-MTHF intravenous bolus administration, an intravenous infusion of 5-FU at a dose of 2,400 mg/m² was initiated. The infusion was paused to administer an intravenous bolus of 6R-MTHF at a dose of 60 mg/m² (completed within 3 minutes). The 5-FU infusion was maintained for 46 hours.

The treatment cycle was repeated once every two weeks for four cycles (8 weeks). After 8 weeks of treatment, the 22 mm lesion was unchanged in size. The treatment produced stable disease and no progression over the 8-week treatment period.

The treatment cycle was repeated once every two weeks for an additional four cycles (8 weeks). The 22 mm lesion was unchanged in size after weeks. The treatment produced no progression of the solid tumors through 16 weeks of treatment.

### EXAMPLE 8--Treatment of Patient Diagnosed with Colorectal Cancer

A 73-year old female (78 kg; 164 cm) was diagnosed with stage IV metastatic colorectal cancer by CT. Colorectal adenocarcinoma was verified by biopsy. One solid tumor (baseline (prior to treatment) CT measurement: 72 mm) was identified in the liver.

The patient received first-line treatment comprising the administration of bevacizumab, oxaliplatin, 5-FU and 6R-MTHF as a second-line therapy. Bevacizumab was administered at a dose of 5 mg/kg by intravenous infusion over 30 to 90 minutes. Following administration of bevacizumab, oxaliplatin was administered at a dose of 85 mg/m² by intravenous infusion between 15 and 120 minutes. Following oxaliplatin administration, 5-fluorouracil (5-FU) at a dose of 400 mg/m² was administered by intravenous bolus within 5 minutes. Thirty minutes after administration of the 5-FU intravenous bolus, 6R-MTHF at a dose of 60 mg/m² was administered by intravenous bolus (completed within 3 minutes). About 5 minutes after completion of the 6R-MTHF intravenous bolus administration, an intravenous infusion of 5-FU at a dose of 2,400 mg/m² was initiated. The infusion was paused to administer an intravenous bolus of 6R-MTHF at a dose of 60 mg/m² (completed within 3 minutes). The 5-FU infusion was maintained for 46 hours.

The treatment cycle was repeated once every two weeks for four cycles (8 weeks). After 8 weeks of treatment, the 72 mm lesion was reduced in size to 59 mm. The treatment produced stable disease and no progression of the solid tumors over the 8-week treatment period.

The treatment cycle was repeated once every two weeks for an additional four cycles (8 weeks). The 59 mm lesion was reduced in size to 53 mm after 16 weeks. The treatment produced stable disease and no progression of the solid tumors through 16 weeks of treatment.

### EXAMPLE 9--Treatment of Patient Diagnosed with Colorectal Cancer

A 57-year old female (77 kg; 160 cm) was diagnosed with stage IV metastatic colorectal cancer by biopsy. One solid tumor (baseline (prior to treatment) CT measurement: 57 mm) was identified in the abdomen.

The patient received second-line treatment comprising the administration of bevacizumab, oxaliplatin, 5-FU and 6R-MTHF as a second-line therapy. Bevacizumab was administered at a dose of 5 mg/kg by intravenous infusion over 30 to 90 minutes. Following administration of bevacizumab, oxaliplatin was administered at a dose of 85 mg/m² by intravenous infusion between 15 and 120 minutes. Following oxaliplatin administration, 5-fluorouracil (5-FU) at a dose of 400 mg/m² was administered by intravenous bolus within 5 minutes. Thirty minutes after administration of the 5-FU intravenous bolus, 6R-MTHF at a dose of 60 mg/m² was administered by intravenous bolus (completed within 3 minutes). About 5 minutes after completion of the 6R-MTHF intravenous bolus administration, an intravenous infusion of 5-FU at a dose of 2,400 mg/m² was initiated. The infusion was paused to administer an intravenous bolus of 6R-MTHF at a dose of 60 mg/m² (completed within 3 minutes). The 5-FU infusion was maintained for 46 hours.

The treatment cycle was repeated once every two weeks for four cycles (8 weeks). After 8 weeks of treatment, the 57 mm lesion was decreased in size to 56 mm. The treatment produced stable disease and no progression of the solid tumors over the 8-week treatment period.

The treatment cycle was repeated once every two weeks for an additional four cycles (8 weeks). The 56 mm lesion was 57 mm after 16 weeks. The treatment produced stable disease and no progression of the solid tumors over the 16-week treatment period.

### EXAMPLE 10--Treatment of Patient Diagnosed with Colorectal Cancer

A 60-year old female (66 kg; 168 cm) was diagnosed with stage IV metastatic colorectal cancer by biopsy. One solid tumor (baseline (prior to treatment) CT measurement: 250 mm) was identified in the abdomen.

The patient received second-line treatment comprising the administration of bevacizumab, oxaliplatin, 5-FU and 6R-MTHF as a second-line therapy. Bevacizumab was administered at a dose of 5 mg/kg by intravenous infusion over 30 to 90 minutes. Following administration of bevacizumab, oxaliplatin was administered at a dose of 85 mg/m² by intravenous infusion between 15 and 120 minutes. Following oxaliplatin administration, 5-fluorouracil (5-FU) at a dose of 400 mg/m² was administered by intravenous bolus within 5 minutes. Thirty minutes after administration of the 5-FU intravenous bolus, 6R-MTHF at a dose of 60 mg/m² was administered by intravenous bolus (completed within 3 minutes). About 5 minutes after completion of the 6R-MTHF intravenous bolus administration, an intravenous infusion of 5-FU at a dose of 2,400 mg/m² was initiated. The infusion was paused to administer an intravenous bolus of 6R-MTHF at a dose of 60 mg/m² (completed within 3 minutes). The 5-FU infusion was maintained for 46 hours.

The treatment cycle was repeated once every two weeks for four cycles (8 weeks). After 8 weeks of treatment, the 250 mm lesion was reduced in size to 188 mm. The treatment produced stable disease and no progression of the solid tumors over the 8-week treatment period.

The treatment cycle was repeated once every two weeks for an additional four cycles (8 weeks). The 188 mm lesion was unchanged in size after 16 weeks. The treatment produced stable disease and no progression of the solid tumors over the 16-week treatment period.

### EXAMPLE 11--Treatment of Patient Diagnosed with Colorectal Cancer

A 69-year old female (84.5 kg; 163 cm) was diagnosed with stage IV metastatic colorectal cancer by CT. Colorectal adenocarcinoma was verified by biopsy. One solid tumor (baseline (prior to treatment) CT measurement: 77 mm) was identified in the liver.

The patient received first-line treatment comprising the administration of bevacizumab, oxaliplatin, 5-FU and 6R-MTHF as a second-line therapy. Bevacizumab was administered at a dose of 5 mg/kg by intravenous infusion over 30 to 90 minutes. Following administration of bevacizumab, oxaliplatin was administered at a dose of 85 mg/m² by intravenous infusion between 15 and 120 minutes. Following oxaliplatin administration, 5-fluorouracil (5-FU) at a dose of 400 mg/m² was administered by intravenous bolus within 5 minutes. Thirty minutes after administration of the 5-FU intravenous bolus, 6R-MTHF at a dose of 60 mg/m² was administered by intravenous bolus (completed within 3 minutes). About 5 minutes after completion of the 6R-MTHF intravenous bolus administration, an intravenous infusion of 5-FU at a dose of 2,400 mg/m² was initiated. The infusion was paused to administer an intravenous bolus of 6R-MTHF at a dose of 60 mg/m² (completed within 3 minutes). The 5-FU infusion was maintained for 46 hours.

The treatment cycle was repeated once every two weeks for four cycles (8 weeks). After 8 weeks of treatment, the 77 mm lesion increased in size to 89 mm.

### EXAMPLE 12--Treatment of Patient Diagnosed with Colorectal Cancer

A 71-year old female (115 kg; 175 cm) was diagnosed with stage IV metastatic colorectal cancer by CT. Colorectal adenocarcinoma was verified by biopsy. One solid tumor (baseline (prior to treatment) CT measurement: 26 mm) was identified in the lung.

The patient received second-line treatment comprising the administration of bevacizumab, oxaliplatin, 5-FU and 6R-MTHF as a second-line therapy. Bevacizumab was administered at a dose of 5 mg/kg by intravenous infusion over 30 to 90 minutes. Following administration of bevacizumab, oxaliplatin was administered at a dose of 85 mg/m² by intravenous infusion between 15 and 120 minutes. Following oxaliplatin administration, 5-fluorouracil (5-FU) at a dose of 400 mg/m² was administered by intravenous bolus within 5 minutes. Thirty minutes after administration of the 5-FU intravenous bolus, 6R-MTHF at a dose of 60 mg/m² was administered by intravenous bolus (completed within 3 minutes). About 5 minutes after completion of the 6R-MTHF intravenous bolus administration, an intravenous infusion of 5-FU at a dose of 2,400 mg/m² was initiated. The infusion was paused to administer an intravenous bolus of 6R-MTHF at a dose of 60 mg/m² (completed within 3 minutes). The 5-FU infusion was maintained for 46 hours.

The treatment cycle was repeated once every two weeks for four cycles (8 weeks). After 8 weeks of treatment, the 26 mm lesion increased in size to 30 mm. The treatment produced stable disease over the 8-week treatment period.

## Claims

1. [6R]-5,10-methylene-tetrahydrofolate (6R-MTHF) for use in the treatment in a human patient diagnosed with colorectal cancer or metastatic colorectal cancer, which treatment comprises the following steps of:
a. administering to said patient a pharmaceutical composition comprising bevacizumab at a dose of 5 mg/kg bevacizumab by intravenous infusion;
b. followed by administering to said patient a pharmaceutical composition comprising oxaliplatin at a dose of 85 mg/m² oxaliplatin by intravenous infusion;
c. followed by administering to said patient a pharmaceutical composition comprising 5-fluorouracil (5-FU) at a dose of 400 mg/m² 5-FU by intravenous bolus;
d. followed by administering to said patient a pharmaceutical composition comprising 6R-MTHF, at a dose of 60 mg/m² 6R-MTHF by intravenous bolus;
e. followed by administering to said patient a pharmaceutical composition comprising 5-FU at a dose of 2,400 mg/m² 5-FU by intravenous infusion over 46 hours; and
f. administering to said patient a pharmaceutical composition comprising 6R-MTHF at a dose of 60 mg/m² 6R-MTHF by intravenous bolus.

2. 6R-MTHF for use in the treatment in a human patient according to claim 1, wherein the intravenous bolus administration of step (d) follows completion of the intravenous bolus administration of step (c) by up to 120 minutes.

3. 6R-MTHF for use in the treatment in a human patient according to claim 1 or 2, wherein the intravenous bolus administration of step (f) follows initiation of the intravenous infusion of step (e) by up to 120 minutes.

4. 6R-MTHF for use in the treatment in a human patient according to any one of claim 1-3, wherein the intravenous infusion of step (e) is paused during the intravenous bolus administration of step (f).

5. 6R-MTHF for use in the treatment in a human patient according to any one of claim 1-4, wherein the intravenous bolus administration of step (f) is completed within 60 minutes of the initiation of step (d).

6. 6R-MTHF for use in the treatment in a human patient according to any one of claim 1-5, wherein the intravenous bolus administration of step (f) follows initiation of step (c) by up to 180 minutes.

7. 6R-MTHF for use in the treatment in a human patient according to any one of claim 1-6, wherein the intravenous bolus administration of step (d) and step (f) are completed within 10 minutes.

8. 6R-MTHF for use in the treatment in a human patient according to any one of claim 1-7, wherein steps (a) to (f) are repeated every 2 weeks.

9. 6R-MTHF for use in the treatment in a human patient according to any one of the preceding claims, wherein the colorectal cancer is colorectal adenocarcinoma.

10. 6R-MTHF for use in the treatment in a human patient according to any one of the preceding claims, wherein the 6R-MTHF is provided as a pharmaceutically acceptable salt.

11. 6R-MTHF for use in the treatment in a human patient according to claim 10, wherein the pharmaceutically acceptable salt of 6R-MTHF is provided as a lyophilizate.

12. 6R-MTHF for use in the treatment in a human patient according to claim 11, wherein the lyophilizate of 6R-MTHF is prepared from 6R-MTHF hemisulfate salt.

13. 6R-MTHF for use in the treatment in a human patient according to any one of claim 11 or 12, wherein the lyophilizate is reconstituted in water.

14. 6R-MTHF for use in the treatment in a human patient according to claim 13, wherein the reconstituted lyophilizate is isotonic and has a pH of between 7.0 and 9.0.

15. 6R-MTHF for use in the treatment in a human patient according to any one of the preceding claims, wherein the 6R-MTHF has a diastereomeric purity of at least 98% d.e.

16. 6R-MTHF for use in the treatment in a human patient according to any one of the preceding claims, wherein the patient has not received any prior cancer treatment.

## Patentansprüche

1. [6R]-5,10-Methylentetrahydrofolat (6R-MTHF) zur Verwendung bei der Behandlung eines menschlichen Patienten, bei dem Kolorektalkrebs oder metastatischer Kolorektalkrebs diagnostiziert wurde, wobei die Behandlung die folgenden Schritte umfasst:
a. die Verabreichung einer Bevacizumab in einer Dosis von 5 mg Bevacizumab/kg umfassenden pharmazeutischen Zusammensetzung durch intravenöse Infusion an den Patienten,
b. gefolgt von der Verabreichung einer Oxaliplatin in einer Dosis von 85 mg Oxaliplatin/m² umfassenden pharmazeutischen Zusammensetzung durch intravenöse Infusion an den Patienten,
c. gefolgt von der Verabreichung einer 5-Fluoruracil (5-FU) in einer Dosis von 400 mg 5-FU/m² umfassenden pharmazeutischen Zusammensetzung durch intravenösen Bolus an den Patienten,
d. gefolgt von der Verabreichung einer 6R-MTHF in einer Dosis von 60 mg 6R-MTHF/m² umfassenden pharmazeutischen Zusammensetzung durch intravenösen Bolus an den Patienten,
e. gefolgt von der Verabreichung einer 5-FU in einer Dosis von 2400 mg 5-FU/m² umfassenden pharmazeutischen Zusammensetzung durch intravenöse Infusion über 46 Stunden an den Patienten, und
f. die Verabreichung einer 6R-MTHF in einer Dosis von 60 mg 6R-MTHF/m² umfassenden pharmazeutischen Zusammensetzung durch intravenösen Bolus an den Patienten.

2. 6R-MTHF zur Verwendung bei der Behandlung eines menschlichen Patienten nach Anspruch 1, wobei die intravenöse Bolusverabreichung von Schritt (d) innerhalb von bis zu 120 Minuten nach Abschluss der intravenösen Bolusverabreichung von Schritt (c) erfolgt.

3. 6R-MTHF zur Verwendung bei der Behandlung eines menschlichen Patienten nach Anspruch 1 oder 2, wobei die intravenöse Bolusverabreichung von Schritt (f) innerhalb von bis zu 120 Minuten nach Beginn der intravenösen Infusion von Schritt (e) erfolgt.

4. 6R-MTHF zur Verwendung bei der Behandlung eines menschlichen Patienten nach einem der Ansprüche 1-3, wobei die intravenöse Infusion von Schritt (e) während der intravenösen Bolusverabreichung von Schritt (f) angehalten wird.

5. 6R-MTHF zur Verwendung bei der Behandlung eines menschlichen Patienten nach einem der Ansprüche 1-4, wobei die intravenöse Bolusverabreichung von Schritt (f) innerhalb von 60 Minuten nach Beginn von Schritt (d) abgeschlossen ist.

6. 6R-MTHF zur Verwendung bei der Behandlung eines menschlichen Patienten nach einem der Ansprüche 1-5, wobei die intravenöse Bolusverabreichung von Schritt (f) innerhalb von bis zu 180 Minuten nach Beginn von Schritt (c) erfolgt.

7. 6R-MTHF zur Verwendung bei der Behandlung eines menschlichen Patienten nach einem der Ansprüche 1-6, wobei die intravenösen Bolusverabreichungen von Schritt (d) und Schritt (f) innerhalb von 10 Minuten abgeschlossen sind.

8. 6R-MTHF zur Verwendung bei der Behandlung eines menschlichen Patienten nach einem der Ansprüche 1-7, wobei die Schritte (a) bis (f) alle 2 Wochen wiederholt werden.

9. 6R-MTHF zur Verwendung bei der Behandlung eines menschlichen Patienten nach einem der vorhergehenden Ansprüche, wobei es sich bei dem Kolorektalkrebs um Kolorektaladenokarzinom handelt.

10. 6R-MTHF zur Verwendung bei der Behandlung eines menschlichen Patienten nach einem der vorhergehenden Ansprüche, wobei das 6R-MTHF als ein pharmazeutisch unbedenkliches Salz bereitgestellt wird.

11. 6R-MTHF zur Verwendung bei der Behandlung eines menschlichen Patienten nach Anspruch 10, wobei das pharmazeutisch unbedenkliche Salz von 6R-MTHF als ein Lyophilisat bereitgestellt wird.

12. 6R-MTHF zur Verwendung bei der Behandlung eines menschlichen Patienten nach Anspruch 11, wobei das Lyophilisat von 6R-MTHF aus 6R-MTHF-Hemisulfatsalz hergestellt wird.

13. 6R-MTHF zur Verwendung bei der Behandlung eines menschlichen Patienten nach Anspruch 11 oder 12, wobei das Lyophilisat in Wasser rekonstituiert wird.

14. 6R-MTHF zur Verwendung bei der Behandlung eines menschlichen Patienten nach Anspruch 13, wobei das rekonstituierte Lyophilisat isotonisch ist und einen pH-Wert zwischen 7,0 und 9,0 aufweist.

15. 6R-MTHF zur Verwendung bei der Behandlung eines menschlichen Patienten nach einem der vorhergehenden Ansprüche, wobei das 6R-MTHF eine Diastereoisomerenreinheit von mindestens 98 % d.e. aufweist.

16. 6R-MTHF zur Verwendung bei der Behandlung eines menschlichen Patienten nach einem der vorhergehenden Ansprüche, wobei der Patient zuvor noch keine Krebsbehandlung erhalten hat.

## Revendications

1. [6R]-5,10-méthylène-tétrahydrofolate (6R-MTHF) destiné à être utilisé dans le traitement chez un patient humain ayant été diagnostiqué avec un cancer colorectal ou un cancer colorectal métastatique, lequel traitement comprend les étapes suivantes :
a. administrer au dit patient une composition pharmaceutique comprenant du bévacizumab à une dose de 5 mg/kg de bévacizumab par perfusion intraveineuse ;
b. suivie par une administration au dit patient d'une composition pharmaceutique comprenant de l'oxaliplatine à une dose de 85 mg/m² d'oxaliplatine par perfusion intraveineuse ;
c. suivie par une administration au dit patient d'une composition pharmaceutique comprenant du 5-fluoro-uracile (5-FU) à une dose de 400 mg/m² de 5-FU par bolus intraveineux ;
d. suivie par une administration au dit patient d'une composition pharmaceutique comprenant du 6R-MTHF à une dose de 60 mg/m² de 6R-MTHF par bolus intraveineux ;
e. suivie par une administration au dit patient d'une composition pharmaceutique comprenant du 5-FU (5-FU) à une dose de 2 400 mg/m² de 5-FU par perfusion intraveineuse sur 46 heures ; et
f. suivie par une administration au dit patient d'une composition pharmaceutique comprenant du 6R-MTHF à une dose de 60 mg/m² de 6R-MTHF par bolus intraveineux.

2. 6R-MTHF destiné à être utilisé dans le traitement chez un patient humain selon la revendication 1, dans lequel l'administration du bolus intraveineux de l'étape (d) intervient jusqu'à 120 minutes après la fin de l'administration du bolus intraveineux de l'étape (c).

3. 6R-MTHF destiné à être utilisé dans le traitement chez un patient humain selon les revendications 1 ou 2, dans lequel l'administration du bolus intraveineux de l'étape (f) intervient jusqu'à 120 minutes après le début de la perfusion intraveineuse de l'étape (e).

4. 6R-MTHF destiné à être utilisé dans le traitement chez un patient humain selon une quelconque revendication des 1-3, dans lequel la perfusion intraveineuse de l'étape (e) est mise en pause pendant l'administration du bolus intraveineux de l'étape (f).

5. 6R-MTHF destiné à être utilisé dans le traitement chez un patient humain selon une quelconque revendication des 1-4, dans lequel l'administration du bolus intraveineux de l'étape (f) est achevée dans les 60 minutes suivant le début de l'étape (d).

6. 6R-MTHF destiné à être utilisé dans le traitement chez un patient humain selon une quelconque revendication des 1-5, dans lequel l'administration du bolus intraveineux de l'étape (f) intervient jusqu'à 180 minutes après le début de l'étape (c).

7. 6R-MTHF destiné à être utilisé dans le traitement chez un patient humain selon une quelconque revendication des 1-6, dans lequel l'administration du bolus intraveineux de l'étape (d) et de l'étape (f) est achevée en 10 minutes.

8. 6R-MTHF destiné à être utilisé dans le traitement chez un patient humain selon une quelconque revendication des 1-7, dans lequel les étapes (a) à (f) sont répétées toutes les 2 semaines.

9. 6R-MTHF destiné à être utilisé dans le traitement chez un patient humain selon l'une quelconque des revendications précédentes, dans lequel le cancer colorectal est un adénocarcinome colorectal.

10. 6R-MTHF destiné à être utilisé dans le traitement chez un patient humain selon l'une quelconque des revendications précédentes, le 6R-MTHF étant fourni sous forme d'un sel acceptable d'un point de vue pharmaceutique.

11. 6R-MTHF destiné à être utilisé dans le traitement chez un patient humain selon la revendication 10, dans lequel le sel acceptable d'un point de vue pharmaceutique du 6R-MTHF est fourni sous forme d'un lyophilisat.

12. 6R-MTHF destiné à être utilisé dans le traitement chez un patient humain selon la revendication 11, dans lequel le lyophilisat du 6R-MTHF est préparé à partir d'un sel d'hémi-sulfate de 6R-MTHF.

13. 6R-MTHF destiné à être utilisé dans le traitement chez un patient humain selon une quelconque des revendications 11 ou 12, dans lequel le lyophilisat est reconstitué dans de l'eau.

14. 6R-MTHF destiné à être utilisé dans le traitement chez un patient humain selon la revendication 13, dans lequel le lyophilisat reconstitué est isotonique et a un pH compris entre 7,0 et 9,0.

15. 6R-MTHF destiné à être utilisé dans le traitement chez un patient humain selon l'une quelconque des revendications précédentes, le 6R-MTHF ayant une pureté diastéréomérique d'au moins 98% e.d.

16. 6R-MTHF destiné à être utilisé dans le traitement chez un patient humain selon l'une quelconque des revendications précédentes, dans lequel le patient n'a reçu aucun traitement contre le cancer au préalable.
